# NEUE EUROPÄISCHE PATENTSCHRIFT

(11) **EP 3 213 789 B2**
(45) Veröffentlichungstag und Bekanntmachung des Hinweises auf die Entscheidung über den Einspruch: **12.01.2022**
(45) Hinweis auf die Patenterteilung: 01.08.2018
(21) Anmeldenummer: 16158227.5
(22) Anmeldetag: 02.03.2016
(51) Int. Cl.: A61M 16/20, F16K 15/14, A61M 15/00

(54) **VENTIL**
VALVE
SOUPAPE

(43) Veröffentlichungstag der Anmeldung: 06.09.2017
(73) Patentinhaber: PARI GmbH Spezialisten für effektive Inhalation, 82319 Starnberg (DE)
(72) Erfinder: Krüger, Ulf, 80799 München (DE)
(74) Vertreter: Hoffmann Eitle

(56) Entgegenhaltungen:
- EP-A1- 2 014 325
- EP-A2- 1 077 339
- EP-A2- 2 008 678
- US-A- 3 504 699
- US-A- 3 504 699
- US-A1- 2004 069 360
- US-A1- 2005 187 524
- US-A1- 2011 108 139
- US-B1- 7 445 028

## Beschreibung

Die Erfindung betrifft ein Ventil für eine Inhaliervorrichtung.

Ventile für Inhaliervorrichtungen sind im Stand der Technik bekannt und beispielsweise in EP 2 008 678 A2 beschrieben. EP 2 008 678 A2 beschreibt ein einteiliges Zweiwegeventilsystem, das Inhalation und Exhalation mit einem einzigen Ventil ermöglicht. Das Ventil hat eine Basis, ein erstes Ventilelement, das in einer Ausführungsform eine Entenschnabelform hat, und ein zweites Ventilelement, das in einer Ausführungsform gestaltet ist wie eine drehbare Klappe.

Ventile für Inhaliervorrichtungen sollen im Gebrauch aus jedem Zustand heraus einen geöffneten und einen geschlossenen oder zumindest nahezu geschlossenen Zustand einnehmen können. Dazu sollten mögliche Verformungen des Ventils eingeschränkt sein. Dies führt zu Einschränkungen bei der Auswahl von geeigneten Geometrien und Werkstoffen für das Ventil.

Verschiedene Ventile sind aus der US 2004/0069360 A1, der US 2005/0187524 A1, der US 7 445 028 B1, der US 2011/108139 A1 und der EP 1 077 339 A2 bekannt.

Aufgabe der Erfindung ist es daher, ein Ventil bereitzustellen, das eine zuverlässige Ventilfunktion aufweist und bei dem trotzdem eine große Gestaltungsfreiheit im Hinblick auf Geometrien und Werkstoffe besteht.

Die Aufgabe wird gelöst durch ein Ventil für eine Inhaliervorrichtung mit einem Innenvolumen, das zumindest teilweise von einer Ventilwand, einer permanenten Öffnung sowie einem Ventilfunktionsbereich begrenzt ist, wobei der Ventilfunktionsbereich eingerichtet ist, unterhalb einer Öffnungsdruckdifferenz zumindest nahezu geschlossen zu sein, und oberhalb einer Öffnungsdruckdifferenz geöffnet zu sein, so dass oberhalb der Öffnungsdruckdifferenz ein Fluid durch die permanente Öffnung in das Innenvolumen hinein und aus dem Ventilfunktionsbereich heraus strömen kann, wobei das Ventil einen Umstülpschutz aufweist, der eingerichtet ist, ein Umstülpen der Ventilwand zu erschweren oder zu verhindern.

Das Ventil weist zweckmäßigerweise ein durchströmbares Innenvolumen auf und ist ein Schnabelventil.

Das Schnabelventil ist vorzugsweise ein einteiliges gegossenes Ventil. Das Schnabelventil ist vorzugsweise eingerichtet, bei niedrigen Druckunterschieden zuverlässig einen Rückfluss zu verhindern. Zweckmäßigerweise weist ein Schnabelventil unterhalb einer Öffnungsdruckdifferenz einen Schlitz als Ventilfunktionsbereich auf, der eingerichtet ist, oberhalb einer Öffnungsdruckdifferenz eine Öffnung zu bilden. Zweckmäßigerweise ist ein Schlitz eine längliche Lücke. Der Schlitz ist vorzugsweise von zwei Bereichen der Ventilwand begrenzt, die im zumindest nahezu geschlossenen Zustand aufeinandertreffen ohne zu überlappen. Der Schlitz kann zwischen zwei Bereichen der Ventilwand vorgesehen sein, die im zumindest nahezu geschlossenen Zustand aufeinanderliegen oder nahe aneinander liegen. In einer bevorzugten Ausführungsform liegen im geschlossenen Zustand zwei Bereiche der Ventilwand mit den Innenseiten aneinander. Der Ventilfunktionsbereich ist zweckmäßigerweise eingerichtet, eine Öffnung zu bilden, indem sich die Bereiche der Ventilwand voneinander entfernen.

Ein Kreuzschlitzventil, das vorliegend nicht beansprucht ist, weist als Ventilfunktionsbereich unterhalb einer Öffnungsdruckdifferenz zwei Schlitze auf, die zusammen eine Kreuzform bilden, und ebenfalls eingerichtet sind, oberhalb einer Öffnungsdruckdifferenz eine Öffnung zu bilden.

Bei einem Joker Valve, das vorliegend nicht beansprucht ist, sind als Ventilfunktionsbereich unterhalb einer Offnungsdruckdifferenz drei Schlitze vorhanden, die sich in einem Punkt treffen, so dass von diesem Punkt aus drei Schlitze strahlenförmig nach außen weisen. Auch diese Schlitze sind eingerichtet, oberhalb der Öffnungsdruckdifferenz eine Öffnung zu bilden.

Auch die Schlitze des Kreuzschlitzventils und des Joker Valves sind vorzugsweise von zwei Bereichen der Ventilwand begrenzt, die im zumindest nahezu geschlossenen Zustand stumpf aufeinandertreffenden. Die Schlitze können zwischen Bereichen der Ventilwand vorgesehen sein, die im zumindest nahezu geschlossenen Zustand aufeinanderliegen oder nahe aneinander liegen. In einer bevorzugten Ausführungsform liegen im geschlossenen Zustand Bereiche der Ventilwand mit den Innenseiten aneinander. Der Ventilfunktionsbereich ist zweckmäßigerweise eingerichtet, eine Öffnung zu bilden, indem sich die Bereiche der Ventilwände voneinander entfernen. Vorzugsweise umfasst die Inhaliervorrichtung eine Aerosolerzeugungsvorrichtung. Eine Aerosolerzeugungsvorrichtung weist bevorzugt einen Vernebler, einen Zerstäuber, einen Befeuchter, einen Druckluftvernebler, einen Luftzerstäuber, einen elektronischen Vernebler, einen Ultraschallvernebler, einen elektrohydrodynamischen Vernebler, einen elektrostatischen Vernebler, einen Membranvernebler, einen Vernebler mit einer vibrierenden Membran, einen elektronischen Vernebler mit einer vibrierenden Membran, einen Mesh-Vernebler, einen Düsenvernebler, einen Dosieraerosol-Inhalator (MDI), einen Pulver-Inhalator (DPI) oder eine Kombination davon auf. Der Dosieraerosol-Inhalator weist in einer Ausführungsform einen unter Druck stehenden Kanister mit einem Medikament und einem Treibgas auf. Zweckmäßigerweise ist der Kanister mit einem von Hand bedienbaren Aktuator verbunden. Es ist vorteilhaft, wenn der Dosieraerosol-Inhalator eingerichtet ist, bei der Aktivierung eine bestimmte Medikamentenmenge in Aerosolform abzugeben. In einer Ausführungsform ist die Aerosolerzeugungsvorrichtung für den Einsatz mit Beatmungsvorrichtungen eingerichtet.

Es ist vorteilhaft, wenn die Inhaliervorrichtung eine Vorrichtung zur Bereitstellung von Aerosolen umfasst. Die Vorrichtung zur Bereitstellung von Aerosolen weist bevorzugt eine Inhalierhilfe, einen Spacer oder eine Chamber auf. Vorrichtungen zur Bereitstellung von Aerosolen sind vorzugsweise Vorrichtungen, die zur Verwendung mit Dosieraerosol-Inhalatoren (MDIs) vorgesehen sind. Sie stellen einen Speicherraum bereit, der dazu geeignet ist, Aerosol bevorzugt aus Dosieraerosol-Inhalatoren aufzunehmen, so dass es von Benutzern daraus abgeatmet werden kann. Spacer weisen keine Ein- und Ausatemventile auf, so dass ein Benutzer seine Atmung so koordinieren sollte, dass er nicht in den Spacer ausatmet. Chambers oder Holding Chambers weisen Aus- und bevorzugt auch Einatemventile auf. So kann erreicht werden, dass ein Ausatemstrom nicht in den Raum, in dem sich das Aerosol befindet, geleitet wird. Es kann erreicht werden, dass ein Medikament nur beim Einatmen aus dem Speicherraum heraus gelangen kann. Die Vorrichtung zur Bereitstellung von Aerosolen kann einen Speicherraum zur Aufnahme und Bereitstellung eines Aerosols, eine Düse zur Erzeugung des Aerosols und eine Halterung für einen Medikamentenkanister aufweisen. Dabei ist der Medikamentenkanister dazu vorgesehen, ein Medikament zur Aerosolerzeugung bereitzustellen.

Aerosole sind Gemische aus festen oder flüssigen Schwebeteilchen und einem Gas.

Aerosole sind bevorzugt zur Applikation auf oder in Teile des menschlichen oder tierischen Körpers, wie Haut, Körperhöhlen, Körperöffnungen, Nase, Nasennebenhöhlen, Kieferhöhle, Stirnhöhle, Keilbeinhöhle, Siebbeinzellen, Rachen, Kehlkopf, Luftröhre, Lunge, Stammbronchus, Bronchien, Bronchiolen, Lungenbläschen, Gelenke oder Bauchraum vorgesehen. Aerosole können eingesetzt werden, um Krankheiten von Menschen und Tieren zu diagnostizieren, vorzubeugen, zu therapieren oder um Menschen oder Tiere gegen Krankheiten zu immunisieren. Ein Innenvolumen ist ein Volumen, das zumindest teilweise von einem oder mehreren Bauteilen umschlossen ist. Das Innenvolumen kann eine Öffnung oder mehrere Öffnungen zur Umgebung aufweisen. Das Innenvolumen kann von einem Ventilfunktionsbereich begrenzt sein, der einen geöffneten und einen geschlossenen Zustand einnehmen kann.

Eine Ventilwand weist vorzugsweise einen Kunststoff, besonders bevorzugt ein Elastomer, Silikon oder Gummi auf. Die Ventilwand grenzt zweckmäßigerweise ein Volumen von einer Umgebung ab. Vorzugsweise begrenzt die Ventilwand ein durchströmbares Volumen.

Eine permanente Öffnung eines Ventils ist eine Öffnung, die im Betrieb des Ventils immer geöffnet ist. Es ist vorteilhaft, wenn die permanente Öffnung im Betrieb des Ventils immer die gleiche Größe hat.

Ein Ventilfunktionsbereich ist ein Bereich eines Ventils, der sowohl zumindest nahezu geschlossen als auch geöffnet sein kann. Zweckmäßigerweise ist der Ventilfunktionsbereich eingerichtet, abhängig von der vorliegenden Druckdifferenz zwischen einer Umgebung und einem Innenvolumen zumindest nahezu geschlossen zu sein oder geöffnet zu sein. Es ist vorteilhaft, wenn der Ventilfunktionsbereich oberhalb einer Öffnungsdruckdifferenz geöffnet ist und unterhalb der Öffnungsdruckdifferenz geschlossen ist.

Die Öffnungsdruckdifferenz ist eine vorgegebene Druckdifferenz, oberhalb derer ein Ventilfunktionsbereich geöffnet sein soll und unterhalb derer der Ventilfunktionsbereich geschlossen sein soll.

Ein Fluid umfasst vorzugsweise ein Gas, eine Flüssigkeit oder ein Aerosol.

Umstülpen ist ein Umwenden, derart, dass danach die Außenseite innen und die Innenseite außen ist. Bei einem Ventil kann ein Umstülpen derart ablaufen, dass eine Ventilwand sich von einem Ventilfunktionsbereich aus aufrollt, der Ventilfunktionsbereich durch die permanente Öffnung hindurch tritt und sich die Ventilwand wieder ausrollt. In diesem Zustand kann das Ventil nicht mehr wie vorgesehen öffnen und schließen.

Ein Umstülpschutz ist eine Einrichtung, die geeignet ist, ein Umstülpen des Ventils zu behindern, zu begrenzen oder zu verhindern.

Vorzugsweise umfasst der Umstülpschutz einen Verstärkungsbereich der Ventilwand. Dadurch kann der Umstülpschutz auf besonders einfache Weise bereitgestellt werden.

In einer Ausführungsform ist als Verstärkungsbereich ein Fremdmaterial mit einem Zweikomponentenverfahren in das Ventil integriert worden. Vorzugsweise ist eine Hartkomponente in das Ventil ein- oder angespritzt worden.

Der Verstärkungsbereich des Ventils kann auf einer Innenseite oder einer Außenseite des Ventils vorgesehen sein.

Zweckmäßigerweise weist der Verstärkungsbereich eine Versteifung auf. Die Versteifung kann auf der abgeflachten oder planen Seite eines Schnabelventils vorgesehen sein. Es können Versteifungen im inneren Bereich des Ventils oder von außen vorgesehen sein.

In einer Ausführungsform sind zylindrische Seitenteile eines Schnabelventils mit einem Verstärkungsbereich versehen.

Eine besonders starke Versteifung lässt sich durch die Kombination von Versteifungen an zylindrischen Seitenteilen und abgeflachten Seiten eines Schnabelventils erreichen.

Der Verstärkungsbereich der Ventilwand ist vorzugsweise ein langgestreckter Bereich, der eine größere Länge als Breite aufweist. Dabei erstreckt sich der Verstärkungsbereich von dem Ventilfunktionsbereich oder aus der Nähe des Ventilfunktionsbereichs in Richtung der permanenten Öffnung.

Vorzugsweise ist der Verstärkungsbereich als verdickter Bereich ausgestaltet. Die Dicke des verdickten Bereichs entspricht vorzugsweise dem doppelten bis zwanzigfachen der Dicke der Ventilwand, besonders bevorzugt dem zwei- bis sechsfachen der Dicke der Ventilwand.

Der verdickte Bereich ist vorzugsweise auf einer Innenseite der Ventilwand vorgesehen. In einer Ausführungsform ist der verdickte Bereich auf einer Außenseite der Ventilwand vorgesehen. Es kann vorteilhaft sein, den verdickten Bereich sowohl auf einer Innenseite als auch auf einer Außenseite der Ventilwand vorzusehen.

In einer Ausführungsform ist der Verstärkungsbereich derart ausgestaltet, dass das Ventil seitlich versteift ist. Vorzugsweise ist das Ventil durch Profile versteift. Besonders bevorzugt sind die Profile direkt in das Material hinein konstruiert. Dadurch kann die Biegesteifigkeit besonders gut erhöht werden.

Wenn der Verstärkungsbereich der Ventilwand von der permanenten Öffnung beabstandet ist, kann auf besonders einfache Weise erreicht werden, dass das Öffnen und Schließen des Ventils so ablaufen kann, dass es von dem Verstärkungsbereich der Ventilwand wenig oder gar nicht behindert ist. Das Ventil ist zweckmäßigerweise so ausgelegt, dass es sich beim Öffnen und Schließen vor allem in dem Bereich direkt an der permanenten Öffnung verformt. Der Bereich direkt an der permanenten Öffnung wirkt vorzugsweise als elastisches Gelenk.

Es ist vorteilhaft, wenn der Verstärkungsbereich derart an der Ventilwand vorgesehen ist, dass der Drehpunkt der Seitenwände zur Öffnung des Ventils identisch bleibt. Zweckmäßigerweise wird der untere Bereich des Ventils freigehalten, um den Drehpunkt zu definieren. Dadurch kann erreicht werden, dass das Gelenk frei ist, das Ventil sich leicht öffnet und die Kraft, die zur Öffnung des Ventils erforderlich ist, sich nicht vergrößert.

Der Abstand des Verstärkungsbereichs der Ventilwand von der permanenten Öffnung entspricht vorzugsweise mindestens der Dicke der Ventilwand. Dabei ist der Abstand des Verstärkungsbereichs der Ventilwand von der permanenten Öffnung zweckmäßigerweise maximal so groß, dass gerade noch Platz für den Verstärkungsbereich vorhanden ist. Besonders bevorzugt beträgt der Abstand des Verstärkungsbereichs der Ventilwand von der permanenten Öffnung das ein- bis fünffache der Dicke der Ventilwand.

In einer Ausführungsform weist die Ventilwand einen Funktionswerkstoff auf und der Verstärkungsbereich der Ventilwand weist einen Stützwerkstoff auf. Dabei hat der Stützwerkstoff einen höheren E-Modul als der Funktionswerkstoff. Auf diese Weise lässt sich mit einem vergleichsweise geringen Materialvolumen eine hohe Stützwirkung erreichen. Es ist möglich, die Ventilwand bei einer relativ dünnen Ausgestaltung stark zu stützen.

Das Ventil kann vollständig oder teilweise im Mehrkomponentenspritzgussverfahren, vorzugsweise im Zweikomponentenspritzgussverfahren hergestellt sein.

Vorzugsweise hat der Verstärkungsbereich der Ventilwand mit dem Stützwerkstoff die gleiche Dicke wie die umgebende Ventilwand. So können Schmutzkanten auf einfache Weise vermieden werden. Wenn der Stützwerkstoff auf einer Außenseite der Ventilwand vorgesehen ist, kann der Stützwerkstoff auf besonders einfache Weise auf die Ventilwand aufgebracht werden. Durch eine Anordnung des Stützwerkstoffs auf einer Innenseite der Ventilwand kann eine besonders effektive Stützwirkung erreicht werden.

Ein Funktionswerkstoff weist vorzugsweise einen E-Modul zwischen 0,003 und 5 kN/mm², besonders bevorzugt zwischen 0,01 und 0,1 kN/mm², auf. Ein Stützwerkstoff weist vorzugsweise einen E-Modul zwischen 1 und 500 kN/mm², besonders bevorzugt zwischen 1 und 50 kN/mm², auf.

Der Funktionswerkstoff weist vorzugsweise ein Elastomer, besonders bevorzugt ein thermoplastisches Elastomer, Silikon oder Gummi auf. Der Stützwerkstoff weist vorzugsweise ein Polymer, besonders bevorzugt Polypropylen, Polyethylen, Polycarbonat, Polystyrol, Polyamid, Polyoxymethylen, einen thermoplastischen Kunststoff, einen verstärkten thermoplastischen Kunststoff, einen faserverstärkten thermoplastischen Kunststoff, Naturfasern oder Glasfasern auf.

Wenn der Verstärkungsbereich der Ventilwand an den Ventilfunktionsbereich angrenzt, kann ein Aufrollen der Ventilwand vom Ventilfunktionsbereich aus auf besonders einfache Weise von Anfang an gestoppt werden. In einer bevorzugten Ausführungsform erstreckt sich der Verstärkungsbereich bis zu einem Schlitz des Ventilfunktionsbereichs.

In einer Ausführungsform ist der Ventilfunktionsbereich eingerichtet, unterhalb einer Öffnungsdruckdifferenz einen Schlitz aufzuweisen und der Verstärkungsbereich der Ventilwand ist eingerichtet, unterhalb einer Öffnungsdruckdifferenz an ein Ende des Schlitzes anzugrenzen. So kann ein Einreißen des Schlitzes verhindert oder erschwert werden.

In einer Ausführungsform erstreckt sich der Verstärkungsbereich vorzugsweise von dem Ende des Schlitzes oder aus der Nähe des Endes des Schlitzes auf der kürzesten Strecke oder nahezu auf der kürzesten Strecke in Richtung der permanenten Öffnung. Diese Ausführungsform ist besonders in Verbindung mit einem Schnabelventil zweckmäßig. In einigen Ausführungsformen des Ventils ist der Bereich zwischen dem Ende des Schlitzes und der permanenten Öffnung ein Bereich, der sich beim Öffnen und Schließen des Ventilfunktionsbereichs wenig oder gar nicht verformt. Durch das Vorsehen des Verstärkungsbereichs in diesem Bereich wird das Öffnen und Schließen daher wenig oder gar nicht behindert.

Es ist vorteilhaft, wenn der Ventilfunktionsbereich eingerichtet ist, unterhalb einer Öffnungsdruckdifferenz einen Schlitz aufzuweisen und der Verstärkungsbereich der Ventilwand unterhalb einer Öffnungsdruckdifferenz an die Mitte des Schlitzes angrenzt. Der Verstärkungsbereich erstreckt sich vorzugsweise in Richtung der permanenten Öffnung. Insbesondere bei einem Schnabelventil erstreckt sich der Verstärkungsbereich vorzugsweise von einer Mitte des Schlitzes des Schnabelventils auf der kürzesten Strecke in Richtung der permanenten Öffnung.

Dadurch ist es auf einfache Weise möglich, den Verstärkungsbereich an einer Stelle der Ventilwand vorzusehen, die beim Öffnen und Schließen des Ventilfunktionsbereichs wenig oder gar nicht verformt wird. Dadurch behindert der Verstärkungsbereich das Öffnen und Schließen des Ventilfunktionsbereichs nicht oder nur in sehr geringem Maße. Das Ventil kann daher leichter derart ausgelegt werden, dass es schon bei geringen Druckdifferenzen oder Unterdrücken öffnet.

In einer Ausführungsform weist der Umstülpschutz ein Stützelement oder eine Ventilgeometrie für eine Ventilwand auf. Vorteilhafterweise weist das Stützelement einen Anlagebereich für eine Ventilwand auf. Der Anlagebereich ist zweckmäßigerweise dazu eingerichtet, dass sich ein Bereich der Ventilwand daran anlegen kann. Derart kann eine Bewegung der Ventilwand auf besonders einfache Weise begrenzt werden. Es ist besonders vorteilhaft, wenn der Anlagebereich eine Anlagefläche oder eine Anlagekante ist. So kann das Umstülpen auf besonders einfache Weise verhindert werden, ohne das Öffnen und Schließen zu behindern. Das Stützelement ist vorzugsweise mit einem Ventilträger verbunden, der dazu eingerichtet ist, das Ventil zu halten. Das Stützelement ist vorzugsweise in den Ventilträger integriert.

Das Stützelement weist vorzugsweise einen Stützwerkstoff, zweckmäßigerweise eine Hartkomponente auf. Zweckmäßigerweise ist das Stützelement an einer Innenseite einer Ventilwand angeordnet. Das Stützelement ist vorzugsweise derart angeordnet, dass sich eine Ventilwand beim Öffnen des Ventilfunktionsbereichs an das Stützelement anlegt, wenn die Öffnung einen Durchlasszustand eingenommen hat, in dem ein vorgesehener Durchlass gebildet ist. Vorzugsweise ist keine weitere Bewegung des Ventilfunktionsbereichs in Richtung der permanenten Öffnung möglich, wenn die Ventilwand an dem Stützelement anliegt.

In einer Ausführungsform ist das Stützelement zur abgeflachten Seite eines Schnabelventils hin ausgerichtet. Das Stützelement kann eine durchgängige Strebe aufweisen. In einer Ausführungsform weist das Stützelement die Form einer Antenne auf.

Zweckmäßigerweise ist das Stützelement eingerichtet, unterhalb einer Öffnungsdruckdifferenz an einer Innenseite der Ventilwand anzuliegen. So kann ein Umstülpen besonders präzise begrenzt oder verhindert werden. Da das Stützelement an der Innenseite der Ventilwand anliegt muss sich die Ventilwand nicht zuerst verformen bis sie an dem Stützelement anliegt und der Umstülpprozess begrenzt oder gestoppt werden kann. Es ist daher auf einfache Weise möglich, den Umstülpprozess in einer frühen Phase zu stoppen.

Es ist vorteilhaft, wenn der Umstülpschutz eine Durchlassunterteilungseinrichtung aufweist, die eingerichtet ist, einen Fluidstrom durch das Innenvolumen in mindestens zwei kleinere Fluidströme aufzuteilen. Größere Fremdkörper können ausgefiltert werden. So kann verhindert werden, dass größere Fremdkörper das Ventil passieren. Zweckmäßigerweise weist die Durchlassunterteilungseinrichtung einen Steg auf. In einer Ausführungsform weist die Durchlassunterteilungseinrichtung eine Lochplatte oder ein Netz auf.

Im Folgenden werden die beigefügten Figuren genauer beschrieben.
Figur 1 zeigt ein Schnabelventil mit zwei Schnabelverstärkungen.
Figur 2 zeigt ein Schnabelventil mit vier Schnabelverstärkungen.
Figur 3 zeigt ein Schnabelventil mit einer Schnabelverstärkung.
Figur 4 zeigt ein Schnabelventil mit zwei Schnabelverstärkungen.
Figur 5 zeigt ein Schnabelventil mit vier Schnabelverstärkungen.
Figur 6 zeigt ein Schnabelventil mit zwei Stützstrukturen.
Figur 7 zeigt ein Schnabelventil mit zwei Stützstrukturen und einer Durchlassunterteilungseinrichtung.
Figur 8 zeigt ein Kreuzschlitzventil mit einem faltennahen Verstärkungsbereich, das vorliegend nicht beansprucht ist.
Figur 9 zeigt ein Kreuzschlitzventil mit einem schlitznahen Verstärkungsbereich, das vorliegend nicht beansprucht ist.
Figur 10 zeigt ein Kreuzschlitzventil mit einem faltennahen und einem schlitznahen Verstärkungsbereich, das vorliegend nicht beansprucht ist.
Figur 11 zeigt ein Joker Valve mit drei Joker Verstärkungsbereichen, das vorliegend nicht beansprucht ist.
Figur 12 zeigt ein Joker Valve mit sechs Verstärkungsbereichen, das vorliegend nicht beansprucht ist.

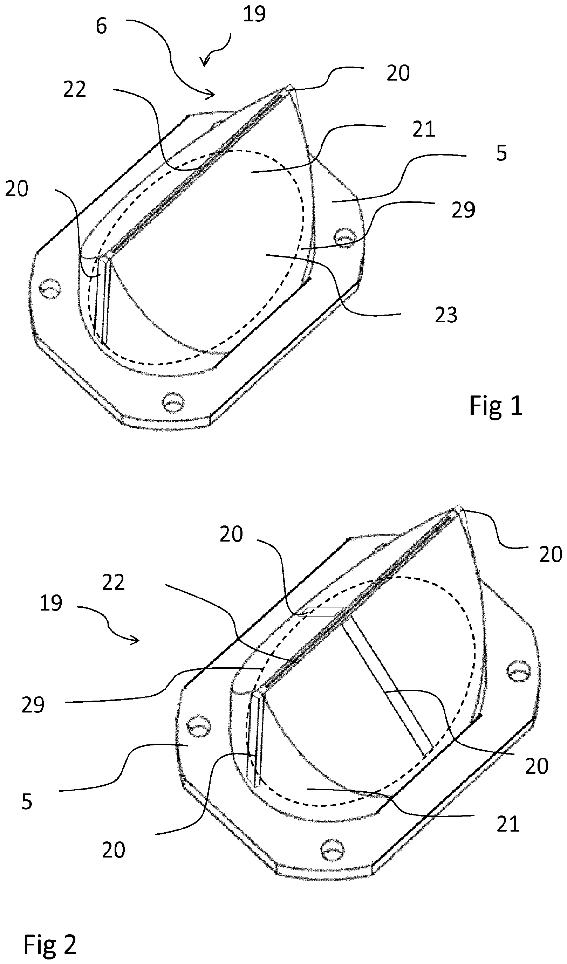

Figur 1 zeigt ein Schnabelventil 19 mit zwei Schnabelverstärkungen 20. Das Schnabelventil 19 weist eine Schnabelventilwand 21 auf, die sich von einem Ventilfuß 5 zu einem Ventilfunktionsbereich 6 erstreckt. Die Schnabelventilwand 21 ist aus einem thermoplastischen Elastomer hergestellt. In dem Ventilfuß 5 ist eine permanente Öffnung 29 vorhanden. Der Ventilfunktionsbereich 6 weist einen Schnabelventilschlitz 22 auf. Wenn der Druck in dem Innenvolumen den Druck in der Umgebung so übersteigt, dass eine Öffnungsdruckdifferenz vorliegt, öffnet sich der Schnabelventilschlitz 22 zu einer Durchlassöffnung. In diesem Zustand kann ein Fluid durch die permanente Öffnung in ein Innenvolumen des Schnabelventils 19 hinein und aus der durch den Schnabelventilschlitz 22 gebildeten Durchlassöffnung heraus strömen.

Das Schnabelventil 19 soll derart ausgeführt sein, dass es sich nicht derart aufrollen und umstülpen kann, dass der Schnabelventilschlitz 22 durch die permanente Öffnung hindurchtritt, wenn im Gebrauch eine Druckdifferenz vorliegt, bei der ein Druck in der Umgebung einen Druck in dem Innenvolumen des Schnabelventils 19 übersteigt.

Um dies zu erreichen, sind zwei Schnabelverstärkungen 20 auf eine Außenseite 23 der Schnabelventilwand 21 aufgeklebt. Die Schnabelverstärkungen 20 sind aus Polyamid hergestellt. Durch ihre Festigkeit verhindern sie ein Aufrollen der Schnabelventilwand 21. Dadurch wird auch ein Umstülpen der Schnabelventilwand 21 wirksam verhindert.

Die Schnabelverstärkungen 20 sind derart auf die Schnabelventilwand 21 aufgeklebt, dass sie jeweils von einem Ende des Schnabelventilschlitzes 22 in Richtung des Ventilfußes 5 verlaufen. Dabei wird ein kleiner Abstand zum Ventilfuß 5 eingehalten. Die Schnabelventilverstärkungen 20 befinden sich so in einem Bereich der Schnabelventilwand 21, der sich beim Öffnen und Schließen des Schnabelventils 19 nicht stark verformt. Der Bereich direkt am Ventilfuß 5 kann wie ein elastisches Gelenk wirken. Die Schnabelventilverstärkungen behindern das Öffnen und Schließen des Schnabelventils 19 daher wenig oder gar nicht.

Figur 2 zeigt ein Schnabelventil 19 mit vier Schnabelverstärkungen 20. Das in Figur 2 gezeigte Schnabelventil 19 ähnelt dem in Figur 1 gezeigten Schnabelventil 19. Ein Unterschied ist, dass die beiden Schnabelverstärkungen 20, die benachbart zu den Enden der Schnabelventilschlitze 22 sind, nicht aufgeklebt sind, sondern aus dem gleichen Material wie die Schnabelventilwand 21 und der Ventilfuß 5 bestehen und zusammen mit den übrigen Teilen des Schnabelventils 19 als Spritzgussteil hergestellt worden sind. Das Material ist ebenfalls ein thermoplastisches Elastomer oder ein Silikon.

Dadurch, dass die Wand durch die Schnabelverstärkungen 20 verdickt ist, erhält sie eine ausreichende Festigkeit, um sich nicht aufzurollen.

Zusätzlich weist das Schnabelventil 19 noch zwei weitere Schnabelventilverstärkungen 20 auf, die sich jeweils von einer Mitte des Schnabelventilschlitzes 22 in Richtung des Ventilfußes 5 erstrecken. Auch diese Schnabelventilverstärkungen 20 erstrecken sich nicht ganz bis zu dem Ventilfuß 5 sondern lassen eine Lücke, damit die Beweglichkeit der Schnabelventilwand 21 nicht eingeschränkt wird. Diese Schnabelventilverstärkungen 20 bestehen ebenfalls aus thermoplastischem Elastomer oder aus Silikon und sind auch zusammen mit dem übrigen Schnabelventil 19 als Spritzgussteil hergestellt worden.

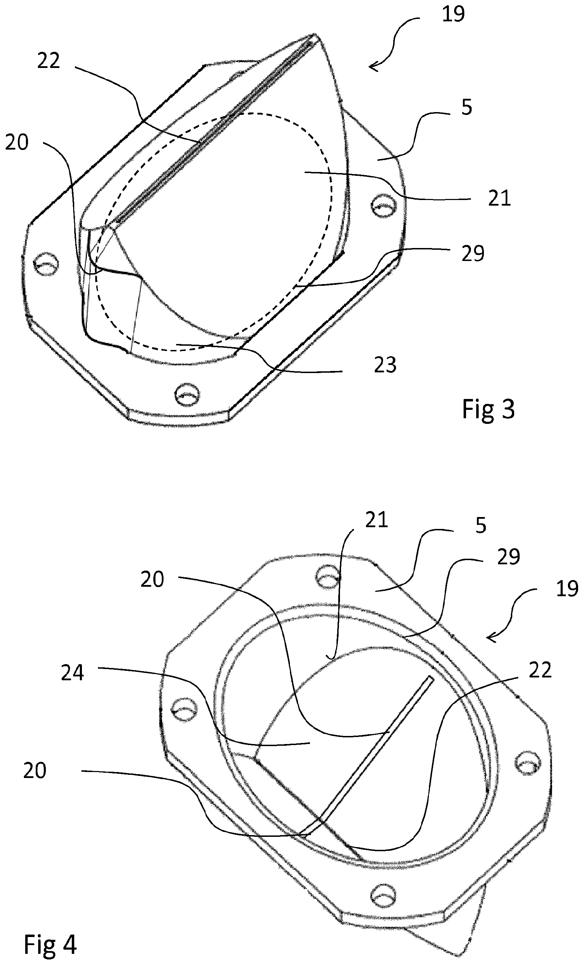

Figur 3 zeigt ein Schnabelventil 19 mit einer Schnabelverstärkung 20. Das Schnabelventil 19 entspricht den in den Figuren 1 und 2 dargestellten Schnabelventilen 19, jedoch ist die Schnabelventilverstärkung 20 anders ausgestaltet. Es ist nur eine Schnabelventilverstärkung 20 vorhanden. Diese erstreckt sich auf einer Außenseite 23 der Schnabelventilwand 21 von einem Ende des Schnabelventilschlitzes 22 in Richtung des Ventilfußes 5. Es ist ebenfalls eine kleine Lücke zwischen der Schnabelventilverstärkung 20 und dem Ventilfuß 5 vorhanden. Die Schnabelventilverstärkung 20 wird zu den Seiten hin, quer zu ihrer Erstreckungsrichtung, dünner, so dass sie fließend in die Schnabelventilwand 21 übergeht.

Figur 4 zeigt ein Schnabelventil 19 mit zwei Schnabelverstärkungen 20. Das in Figur 4 gezeigte Schnabelventil 19 ähnelt dem in Figur 2 gezeigten Schnabelventil 19. Es sind jedoch nur zwei Schnabelventilverstärkungen 20 vorhanden. Diese sind auf einer Innenseite 24 der Schnabelventilwand 21 vorgesehen und erstrecken sich von einer Mitte des Schnabelventilschlitzes 22 in Richtung des Ventilfußes 5. Es ist ebenfalls eine kleine Lücke zwischen der Schnabelventilverstärkung 20 und dem Ventilfuß 5 vorhanden.

Auch bei dem in Figur 4 gezeigten Schnabelventil 19 sind die Schnabelventilverstärkungen 20 eingerichtet, ein Umstülpen des Schnabelventils 19 zu verhindern, ohne das Öffnen und Schließen des Schnabelventils 19 zu behindern.

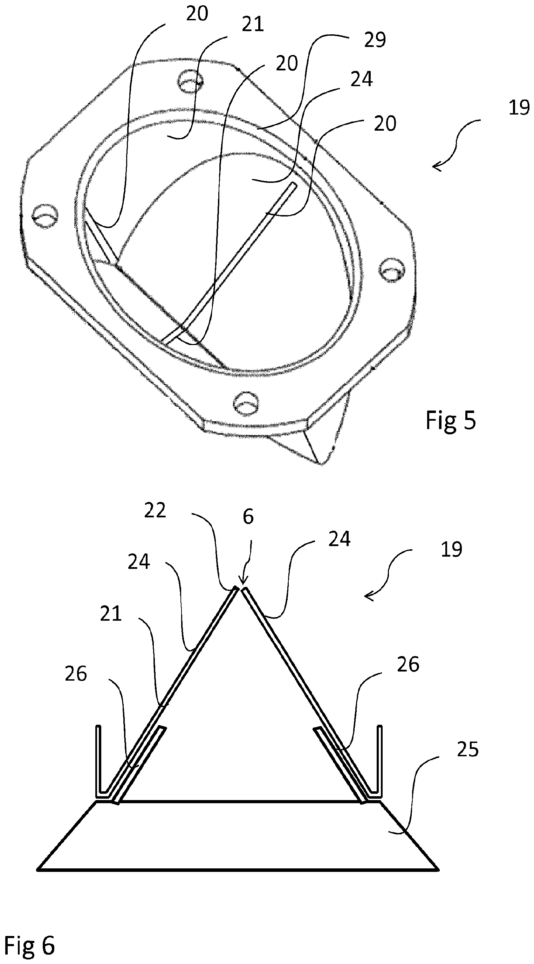

Figur 5 zeigt ein Schnabelventil 19 mit vier Schnabelverstärkungen 20. In Figur 5 sind nur drei der Schnabelverstärkungen 5 sichtbar. Das in Figur 5 gezeigte Schnabelventil 19 ähnelt dem in Figur 4 gezeigten Schnabelventil 19. Es sind jedoch noch zwei zusätzliche Schnabelventilverstärkungen 20 vorhanden, die sich von den Enden des Schnabelventilschlitzes 22 in Richtung des Ventilfußes 5 erstrecken. Diese sind ebenfalls auf der Innenseite 24 der Schnabelventilwand 21 vorgesehen. Auch hier ist eine kleine Lücke zwischen der Schnabelventilverstärkung 20 und dem Ventilfuß 5 vorhanden.

Durch diese beiden zusätzlichen Schnabelventilverstärkungen 20 wird das Öffnen und Schließen des Schnabelventils 19 ebenfalls nicht behindert. Da vier Schnabelventilverstärkungen 20 vorhanden sind, können die einzelnen Schnabelventilverstärkungen 20 dünner oder flexibler ausgestaltet sein als bei dem in Figur 4 gezeigten Schnabelventil und trotzdem die gleiche Wirkung gegen Aufrollen und Umstülpen entfalten.

Figur 6 zeigt ein Schnabelventil 19 mit zwei Stützstrukturen 26. Das Schnabelventil 19 ähnelt den in den Figuren 1 bis 5 gezeigten Schnabelventilen 19, jedoch ist die Schnabelventilwand 21 nicht mit Schnabelventilverstärkungen 20 versehen. Das Schnabelventil 19 ist an einem Ventilträger 25 befestigt, der mit zwei Stützstrukturen 26 versehen ist. Die beiden Stützstrukturen 26 erstrecken sich an der Innenseite 24 der Schnabelventilwand 21 entlang in Richtung des Schnabelventilschlitzes 22.

Dadurch, dass die Stützstrukturen 26 nicht mit der Schnabelventilwand 21 verbunden sind, beeinträchtigen sie das Öffnen und Schließen des Ventilfunktionsbereichs 6 nicht.

Ein Aufrollen und Umstülpen der Schnabelventilwand 21 wird jedoch wirksam verhindert, da die Stützstrukturen 26 eine Anlagekante für eine Schnabelventilwand 21 und eine ausreichende Festigkeit aufweisen, um ein Aufrollen der Schnabelventilwand 21 zu begrenzen.

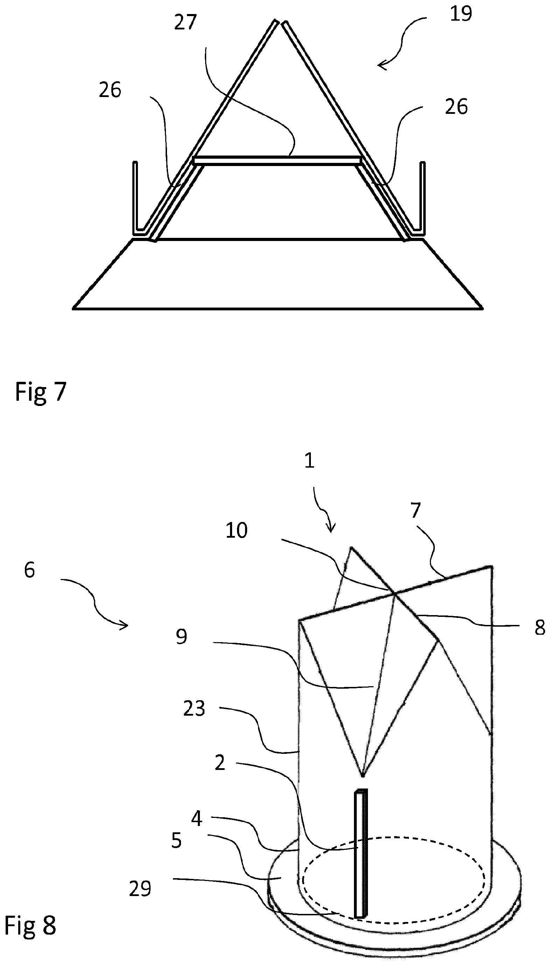

Figur 7 zeigt ein Schnabelventil 19 mit zwei Stützstrukturen 26 und einer Durchlassunterteilungseinrichtung 27. Das in Figur 7 gezeigte Schnabelventil 19 ähnelt dem in Figur 6 gezeigten Schnabelventil 19. Es sind die gleichen Stützstrukturen 26 vorhanden, jedoch sind diese durch eine als Steg 27 ausgebildete Durchlassunterteilungseinrichtung 27 miteinander verbunden.

Der Steg 27 bewirkt, dass der Durchlass durch das Schnabelventil 19 in zwei kleinere Durchlässe unterteilt ist. Dadurch ist die Größe der Körper, die das Ventil passieren können, stärker eingeschränkt.

Figur 8 zeigt ein Kreuzschlitzventil 1 mit einem faltennahen Verstärkungsbereich 2. Das Kreuzschlitzventil 1 weist eine Kreuzschlitzventilwand 4 mit einer zylindrischen Form auf, die an einem Ende in einen Ventilfuß 5 übergeht. In dem Ventilfuß 5 ist eine permanente Öffnung 29 vorgesehen. An dem anderen Ende der zylindrischen Form geht die Kreuzschlitzventilwand 4 in den Ventilfunktionsbereich 6 mit einem ersten Schlitz 7 und einem zweiten Schlitz 8, die kreuzförmig angeordnet sind, über. In dem in Figur 8 gezeigten Zustand sind der erste Schlitz 7 und der zweite Schlitz 8 geschlossen. Es sind vier Falten 9 vorgesehen, die von einem Mittelpunkt 10, in dem der erste Schlitz 7 und der zweite Schlitz 8 sich kreuzen, zu der Kreuzschlitzventilwand 4 verlaufen. Innerhalb der Kreuzschlitzventilwand 4 zwischen der permanenten Öffnung und dem Ventilfunktionsbereich 6 befindet sich ein Innenvolumen. Auf einer Außenseite 23 der Kreuzschlitzventilwand 4 ist der faltennahe Verstärkungsbereich 2 angeordnet. Der faltennahe Verstärkungsbereich 2 erstreckt sich zwischen dem Endpunkt einer Falte 9 auf der Kreuzschlitzventilwand 4 und dem Ventilfuß 5. Dabei ist der faltennahe Verstärkungsbereich 2 sowohl von der Falte 9 als auch von dem Ventilfuß 5 beabstandet. Der Werkstoff, aus dem das Kreuzschlitzventil 1 mit dem faltennahen Verstärkungsbereich 2 hergestellt worden ist, ist Silikon. Das Kreuzschlitzventil 1 mit dem faltennahen Verstärkungsbereich 2 ist einstückig gegossen worden.

Wenn der Druck in dem Innenvolumen den Druck in der Umgebung übersteigt und die Druckdifferenz zwischen Innenvolumen und Umgebung einen festgelegten Grenzwert übersteigt, öffnen sich der erste Schlitz 7 und der zweite Schlitz 8 zu einer Öffnung, so dass ein Gas durch die permanente Öffnung in den Bereich innerhalb der Kreuzschlitzventilwand 4 hinein und aus der Öffnung in dem Ventilfunktionsbereich 6 heraus strömen kann. Dabei verformt sich der Ventilfunktionsbereich 6. Der Bereich der Kreuzschlitzventilwand 4, in dem der faltennahe Verstärkungsbereich 2 angeordnet ist, verformt sich beim Öffnen und Schließen des Ventilfunktionsbereichs 6 wenig oder gar nicht. Der faltennahe Verstärkungsbereich 2 ist von der Falte 9 und dem Ventilfuß 5 beabstandet, weil sich das Kreuzschlitzventil 1 direkt an der Falte 9 und an dem Ventilfuß 5 beim Öffnen und Schließen stärker verformt. Daher beeinträchtigt der faltennahe Verstärkungsbereich 2 das Öffnen und Schließen des Ventilfunktionsbereichs 6 wenig oder gar nicht.

Wenn eine Druckdifferenz vorliegt, bei der der Druck in der Umgebung höher ist als der Druck in dem Innenvolumen, kann es passieren, dass der Ventilfunktionsbereich 6 zu dem Bereich innerhalb der Kreuzschlitzventilwand 4 gedrückt wird. Wenn kein faltennaher Verstärkungsbereich 2 vorhanden wäre, könnte der Ventilfunktionsbereich 6 durch die permanente Öffnung hindurch gelangen, so dass das Kreuzschlitzventil 1 umgestülpt wäre. Bei diesem Vorgang würde das Kreuzschlitzventil 1 über die Kreuzschlitzventilwand 4 abrollen. Bei der in Figur 8 gezeigten Ausführungsform verhindert der faltennahe Verstärkungsbereich 2 das Abrollen und damit das Umstülpen des Kreuzschlitzventils 1.

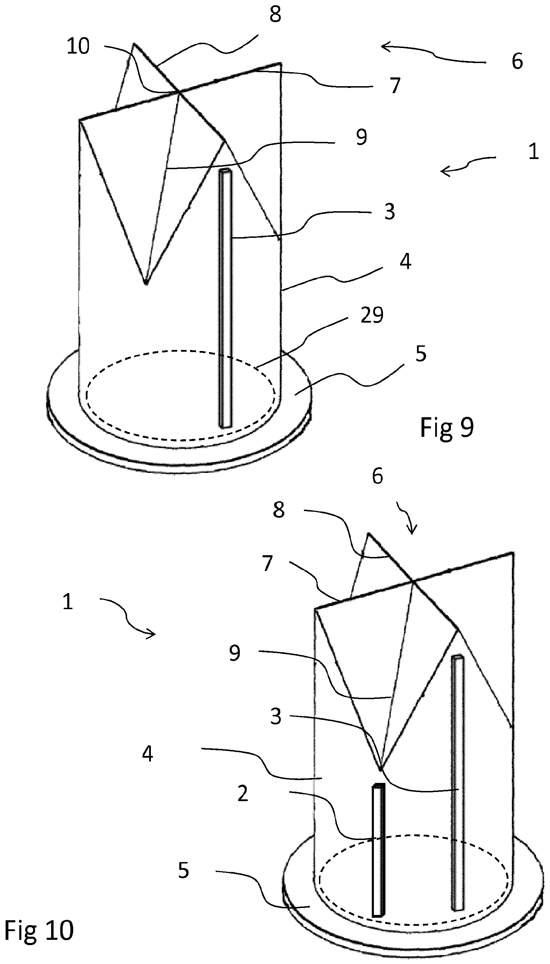

Figur 9 zeigt ein Kreuzschlitzventil 1 mit einem schlitznahen Verstärkungsbereich 3. Das in Figur 9 gezeigte Kreuzschlitzventil 1 ähnelt dem in Figur 8 gezeigten Kreuzschlitzventil 1. Der Unterschied liegt darin, dass der schlitznahe Verstärkungsbereich 3 nicht an eine Falte 9 sondern an den zweiten Schlitz 8 angrenzt, wobei ebenfalls ein Abstand zu dem Schlitz eingehalten ist. Außerdem ist der schlitznahe Verstärkungsbereich 3 bei dem in Figur 9 gezeigten Kreuzschlitzventil 1 nicht zusammen mit dem übrigen Kreuzschlitzventil 1 gegossen worden, sondern auf die Kreuzschlitzventilwand 4 aufgeklebt worden. Das Kreuzschlitzventil 1 ist mit Ausnahme des schlitznahen Verstärkungsbereichs 3 aus Silikon gegossen worden. Der schlitznahe Verstärkungsbereich 3 ist aus Polyamid hergestellt.

Auch der in Figur 9 gezeigte schlitznahe Verstärkungsbereich 3 befindet sich in einem Bereich des Kreuzschlitzventils 1, der sich beim Öffnen und Schließen wenig oder gar nicht verformt. Das Öffnen und Schließen wird daher wenig oder gar nicht beeinträchtigt. Da der schlitznahe Verstärkungsbereich 3 an den zweiten Schlitz 8 angrenzt, kann ein Ausreißen des zweiten Schlitzes 8 an dieser Seite begrenzt werden.

Wenn eine Druckdifferenz vorliegt, bei der der Druck in der Umgebung höher ist als der Druck in dem Innenvolumen, verhindert der in Figur 9 gezeigte schlitznahe Verstärkungsbereich 3 entsprechend dem in Figur 8 gezeigten faltennahen Verstärkungsbereich 2 ein Aufrollen und Umstülpen des Kreuzschlitzventils 1. Der unerwünschte Aufrollprozess wird etwas früher gestoppt als bei dem in Figur 8 gezeigten Kreuzschlitzventil, da der schlitznahe Verstärkungsbereich 3 sich näher an dem Bereich des Funktionsbereichs 5 befindet, der abhängig von der anliegenden Druckdifferenz eine Öffnung aufweist.

Figur 10 zeigt ein Kreuzschlitzventil 1 mit einem faltennahen Verstärkungsbereich 2 und einem schlitznahen Verstärkungsbereich 3. Das Kreuzschlitzventil 1 entspricht den in den Figuren 8 und 9 gezeigten Kreuzschlitzventilen 1. Der faltennahe Verstärkungsbereich 2 entspricht dem in Figur 8 gezeigten faltennahen Verstärkungsbereich 2 und der schlitznahe Verstärkungsbereich 3 entspricht dem in Figur 9 gezeigten schlitznahen Verstärkungsbereich 3.

Durch die Kombination des faltennahen Verstärkungsbereichs 2 mit dem schlitznahen Verstärkungsbereich 3 wird ein noch besserer Schutz gegen Aufrollen und Umstülpen des Kreuzschlitzventils 1 erzielt.

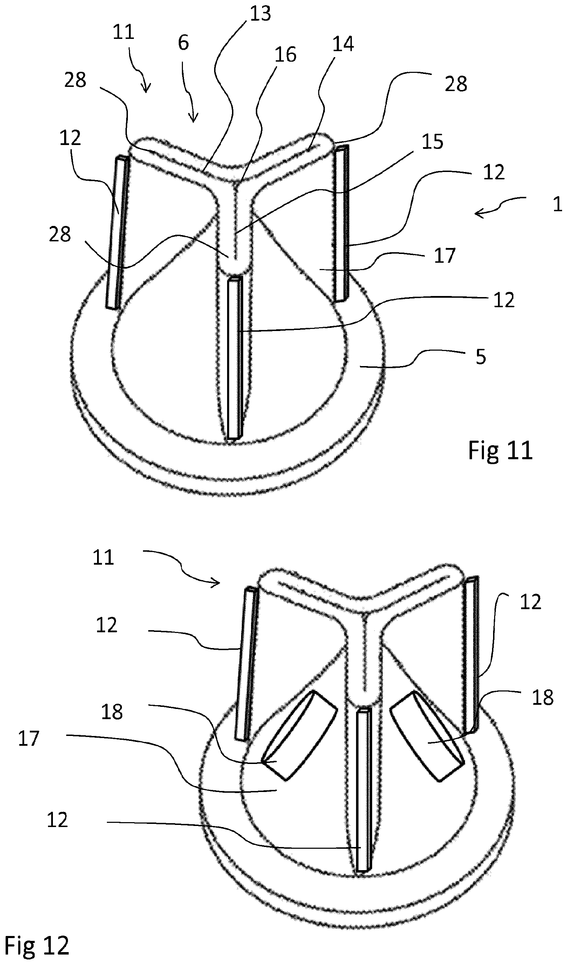

Figur 11 zeigt ein Joker Valve 11 mit drei Joker Verstärkungsbereichen 12. Das Joker Valve 11 weist einen Ventilfuß 5 mit einer nicht gezeigten permanenten Öffnung, eine Joker Ventilwand 17 und einen Ventilfunktionsbereich 6 auf. Die Joker Ventilwand 17 umschließt ein Innenvolumen, das von der permanenten Öffnung und dem Ventilfunktionsbereich 6 begrenzt wird. Der Ventilfunktionsbereich 6 weist drei Joker Schlitze 13, 14 und 15 auf, die sich von einem Joker Mittelpunkt 16 nach außen erstrecken. Im Bereich der Joker Schlitze 13, 14 und 15 bildet die Joker Ventilwand 17 jeweils eine Joker Falte 28, so dass an jedem Joker Schlitz 13, 14 und 15 zwei Joker Ventilwände 17 aufeinander liegen, wenn die Joker Schlitze 13, 14 und 15 geschlossen sind.

Die drei Joker Verstärkungsbereiche 12 verlaufen auf der Joker Ventilwand 17 jeweils von einem Bereich, der einem Ende eines Joker Schlitzes 13, 14 und 15 benachbart ist, in Richtung des Ventilfußes 5. Dabei wird ein Abstand zum Ventilfuß 5 eingehalten. Das Joker Valve 11 ist mit Ausnahme der Joker Verstärkungsbereiche 12 aus Silikon hergestellt. Die Joker Verstärkungsbereiche 12 weisen Polystyrol auf. Das Joker Valve 11 ist im Mehrkomponentenspritzgussverfahren hergestellt worden.

Wenn der Druck im Innenvolumen so ansteigt, dass ein vorgegebener Differenzdruck zwischen dem Innenvolumen und der Umgebung überschritten wird, öffnen sich die Joker Schlitze 13, 14 und 15 zu einer Durchlassöffnung. Da sich die Joker Verstärkungsbereiche 12 in einem Bereich der Joker Ventilwand 17 befinden, der sich beim Öffnen und Schließen des Joker Valves 11 wenig oder gar nicht verformt, wird das Öffnen und Schließen des Joker Valves 11 durch die Joker Verstärkungsbereiche 12 nicht beeinträchtigt.

Wenn der Druck in der Umgebung den Druck im Innenvolumen übersteigt, wird der Ventilfunktionsbereich 6 in Richtung der permanenten Öffnung gedrückt. Die Joker Verstärkungsbereiche 12 sind geeignet, ein Aufrollen und Umstülpen des Joker Valves 11 zu verhindern.

Figur 12 zeigt ein Joker Valve 11 mit sechs Verstärkungsbereichen, davon 3 Joker Verstärkungsbereiche 12 und 3 Ovalverstärkungsbereiche 18. Das in Figur 12 gezeigte Joker Valve 11 ähnelt dem in Figur 11 gezeigten Joker Valve 11. Der Unterschied ist, dass zusätzlich zu den 3 Joker Verstärkungsbereichen 12 die 3 Ovalverstärkungsbereiche 18 vorgesehen sind. Die Ovalverstärkungsbereiche 18 sind außen an der Joker Ventilwand 17 vorgesehen. In der Draufsicht erscheinen sie oval. Die Ovalverstärkungsbereiche 18 sind aus Polystyrol hergestellt und das Joker Valve 11 ist im Mehrkomponentenspritzgussverfahren hergestellt worden.

Die Ovalverstärkungsbereiche 18 bieten einen zusätzlichen Schutz gegen Aufrollen und Umstülpen.

### BEZUGSZEICHENLISTE

- 1: Kreuzschlitzventil
- 2: faltennaher Verstärkungsbereich
- 3: schlitznaher Verstärkungsbereich
- 4: Kreuzschlitzventilwand
- 5: Ventilfuß
- 6: Ventilfunktionsbereich
- 7: erster Schlitz
- 8: zweiter Schlitz
- 9: Falte
- 10: Mittelpunkt
- 11: Joker Valve
- 12: Joker Verstärkungsbereiche
- 13, 14, 15: Joker Schlitz
- 16: Joker Mittelpunkt
- 17: Joker Ventilwand
- 18: Ovalverstärkungsbereich
- 19: Schnabelventil
- 20: Schnabelverstärkung
- 21: Schnabelventilwand
- 22: Schnabelventilschlitz
- 23: Außenseite
- 24: Innenseite
- 25: Ventilträger
- 26: Stützstruktur
- 27: Steg
- 28: Joker Falte
- 29: Permanente Öffnung

## Patentansprüche

1. Ventil (1, 11, 19) für eine Inhaliervorrichtung mit einem Innenvolumen, das zumindest teilweise von einer Ventilwand (4, 17, 21), einer permanenten Öffnung (29) sowie einem Ventilfunktionsbereich (6) begrenzt ist,
wobei der Ventilfunktionsbereich (6) eingerichtet ist, unterhalb einer Öffnungsdruckdifferenz zumindest nahezu geschlossen zu sein, und oberhalb einer Öffnungsdruckdifferenz geöffnet zu sein, so dass oberhalb der Öffnungsdruckdifferenz ein Fluid durch die permanente Öffnung (29) in das Innenvolumen hinein und aus dem Ventilfunktionsbereich (6) heraus strömen kann,
**dadurch gekennzeichnet, dass** das Ventil einen Umstülpschutz aufweist, der eingerichtet ist, ein Umstülpen der Ventilwand (4, 17, 21) zu erschweren oder zu verhindern, und das Ventil ein Schnabelventil ist.

2. Ventil (1, 11, 19) nach Anspruch 1, **dadurch gekennzeichnet, dass** der Umstülpschutz einen Verstärkungsbereich (2, 3, 12, 18, 20) der Ventilwand (4, 17, 21) umfasst.

3. Ventil (1, 11, 19) nach Anspruch 2, **dadurch gekennzeichnet, dass** der Verstärkungsbereich (2, 3, 12, 18, 20) der Ventilwand (4, 17, 21) von der permanenten Öffnung (29) beabstandet ist.

4. Ventil (1, 11, 19) nach Anspruch 2 oder Anspruch 3, **dadurch gekennzeichnet, dass** die Ventilwand (4, 17, 21) einen Funktionswerkstoff aufweist und der Verstärkungsbereich (2, 3, 12, 18, 20) der Ventilwand (4, 17, 21) einen Stützwerkstoff aufweist, der einen höheren E-Modul hat als der Funktionswerkstoff.

5. Ventil (1, 11, 19) nach einem der Ansprüche 2 bis 4, **dadurch gekennzeichnet, dass** der Verstärkungsbereich (2, 3, 12, 18, 20) der Ventilwand (4, 17, 21) an den Ventilfunktionsbereich (6) angrenzt.

6. Ventil (1, 11, 19) nach Anspruch 5, **dadurch gekennzeichnet, dass** der Ventilfunktionsbereich (6) eingerichtet ist, unterhalb einer Öffnungsdruckdifferenz einen Schlitz (7, 8, 13, 14, 15, 22) aufzuweisen und der Verstärkungsbereich (2, 3, 12, 18, 20) der Ventilwand (4, 17, 21) an ein Ende des Schlitzes (7, 8, 13, 14, 15, 22) angrenzt.

7. Ventil (1, 11, 19) nach einem der vorhergehenden Ansprüche, **dadurch gekennzeichnet, dass** der Ventilfunktionsbereich (6) eingerichtet ist, unterhalb einer Öffnungsdruckdifferenz einen Schlitz (7, 8, 13, 14, 15, 22) aufzuweisen und der Verstärkungsbereich (2, 3, 12, 18, 20) der Ventilwand (4, 17, 21) unterhalb einer Öffnungsdruckdifferenz an die Mitte des Schlitzes (7, 8, 13, 14, 15, 22) angrenzt.

8. Ventil (1, 11, 19) nach Anspruch 1, **dadurch gekennzeichnet, dass** der Umstülpschutz ein Stützelement (26) für eine Ventilwand (4, 17, 21) aufweist.

9. Ventil (1, 11, 19) nach Anspruch 8, **dadurch gekennzeichnet, dass** das Stützelement (26) eingerichtet ist, unterhalb einer Öffnungsdruckdifferenz an einer Innenseite (24) der Ventilwand (4, 17, 21) anzuliegen.

10. Ventil (1, 11, 19) nach Anspruch 8 oder Anspruch 9, **dadurch gekennzeichnet, dass** der Umstülpschutz (26) eine Durchlassunterteilungseinrichtung (27) aufweist, die eingerichtet ist, einen Fluidstrom durch das Innenvolumen in mindestens zwei kleinere Fluidströme aufzuteilen.

11. Verwendung des Ventils nach einem der vorhergehenden Ansprüche in einer Inhaliervorrichtung.

12. Inhaliervorrichtung mit einem Ventil nach einem der vorhergehenden Ansprüche 1 bis 10.

## Claims

1. A valve (1, 11, 19) for an inhalation device comprising an interior volume that is delimited at least partially by a valve wall (4, 17, 21), a permanent opening (29) as well as a valve functional region (6), the valve functional region (6) being configured to be at least almost closed below an opening pressure difference and to be open above an opening pressure difference such that above the opening pressure difference, a fluid can flow through the permanent opening (29) into the interior volume and out of the valve functional region (6), **characterised in that** the valve comprises an inversion protector that is configured to impede or to prevent inversion of the valve wall (4, 17, 21) and the valve is a duck beak valve.

2. The valve (1, 11, 19) according to Claim 1, **characterised in that** the inversion protector comprises a reinforcing region (2, 3, 12, 18, 20) of the valve wall (4, 17, 21) .

3. The valve (1, 11, 19) according to Claim 2, **characterised in that** the reinforcing region (2, 3, 12, 18, 20) of the valve wall (4, 17, 21) is spaced apart from the permanent opening (29).

4. The valve (1, 11, 19) according to Claim 2 or Claim 3, **characterised in that** the valve wall (4, 17, 21) comprises a functional material and the reinforcing region (2, 3, 12, 18, 20) of the valve wall (4, 17, 21) comprises a support material which has a higher modulus of elasticity than the functional material.

5. The valve (1, 11, 19) according to any of Claims 2 to 4, **characterised in that** the reinforcing region (2, 3, 12, 18, 20) of the valve wall (4, 17, 21) abuts the valve functional region (6).

6. The valve (1, 11, 19) according to Claim 5, **characterised in that** the valve functional region (6) is configured to have a slit (7, 8, 13, 14, 15, 22) below an opening pressure difference and the reinforcing region (2, 3, 12, 18, 20) of the valve wall (4, 17, 21) abuts an end of the slit (7, 8, 13, 14, 15, 22).

7. The valve (1, 11, 19) according to any of the preceding claims, **characterised in that** the valve functional region (6) is configured to have a slit (7, 8, 13, 14, 15, 22) below an opening pressure difference and the reinforcing region (2, 3, 12, 18, 20) of the valve wall (4, 17, 21) abuts the centre of the slit (7, 8, 13, 14, 15, 22) below an opening pressure difference.

8. The valve (1, 11, 19) according to Claim 1, **characterised in that** the inversion protector comprises a support element (26) for a valve wall (4, 17, 21).

9. The valve (1, 11, 19) according to Claim 8, **characterised in that** the support element (26) is configured to abut an inner side (24) of the valve wall (4, 17, 21) below an opening pressure difference.

10. The valve (1, 11, 19) according to Claim 8 or Claim 9, **characterised in that** the inversion protector (26) comprises a passage dividing device (27) that is configured to divide a flow of fluid through the interior volume into at least two smaller flows of fluid.

11. The use of the valve according to any of the preceding claims in an inhalation device.

12. An inhalation device having a valve according to any of the preceding Claims 1 to 10.

## Revendications

1. Soupape (1, 11, 19) pour un dispositif d'inhalation avec un volume intérieur qui est délimité au moins en partie par une paroi de soupape (4, 17, 21), une ouverture permanente (29) ainsi qu'une zone fonctionnelle de soupape (6), dans laquelle la zone fonctionnelle de soupape (6) est conçue pour être au moins presque fermée au-dessous d'une différence de pression d'ouverture et être ouverte au-dessus d'une différence de pression d'ouverture de telle sorte qu'au-dessus de la différence de pression d'ouverture, un fluide peut, via l'ouverture permanente (29), s'écouler en entrant dans le volume intérieur et en sortant de la zone fonctionnelle de soupape (6),
**caractérisée en ce que** la soupape comporte une protection anti-retournement qui est conçue pour empêcher ou rendre difficile un retournement de la paroi de soupape (4, 17, 21), et la soupape est une soupape à bec.

2. Soupape (1, 11, 19) selon la revendication 1, **caractérisée en ce que** la protection anti-retournement comprend une zone de renfort (2, 3, 12, 18, 20) de la paroi de soupape (4, 17, 21).

3. Soupape (1, 11, 19) selon la revendication 2, **caractérisée en ce que** la zone de renfort (2, 3, 12, 18, 20) de la paroi de soupape (4, 17, 21) est distante de l'ouverture permanente (29).

4. Soupape (1, 11, 19) selon la revendication 2 ou 3, **caractérisée en ce que** la paroi de soupape (4, 17, 21) a un matériau fonctionnel et la zone de renfort (2, 3, 12, 18, 20) de la paroi de soupape (4, 17, 21) a un matériau de soutien, qui présente un plus grand coefficient d'élasticité que le matériau fonctionnel.

5. Soupape (1, 11, 19) selon l'une quelconque des revendications 2 à 4, **caractérisée en ce que** la zone de renfort (2, 3, 12, 18, 20) de la paroi de soupape (4, 17, 21) est adjacente à la zone fonctionnelle de soupape (6).

6. Soupape (1, 11, 19) selon la revendication 5, **caractérisée en ce que** la zone fonctionnelle de soupape (6) est conçue pour présenter une fente (7, 8, 13, 14, 15, 22) au-dessous d'une différence de pression d'ouverture et la zone de renfort (2, 3, 12, 18, 20) de la paroi de soupape (4, 17, 21) est adjacente à une extrémité de la fente (7, 8, 13, 14, 15, 22).

7. Soupape (1, 11, 19) selon l'une quelconque des revendications précédentes, **caractérisée en ce que** la zone fonctionnelle de soupape (6) est conçue pour présenter une fente (7, 8, 13, 14, 15, 22) au-dessous d'une différence de pression d'ouverture et la zone de renfort (2, 3, 12, 18, 20) de la paroi de soupape (4, 17, 21) est adjacente au milieu de la fente (7, 8, 13, 14, 15, 22) au-dessous de la différence de pression d'ouverture.

8. Soupape (1, 11, 19) selon la revendication 1, **caractérisée en ce que** la protection anti-retournement comporte un élément de soutien (26) pour une paroi de soupape (4, 17, 21).

9. Soupape (1, 11, 19) selon la revendication 8, **caractérisée en ce que** l'élément de soutien (26) est conçu pour être contre un côté intérieur (24) de la paroi de soupape (4, 17, 21) au-dessous d'une différence de pression d'ouverture.

10. Soupape (1, 11, 19) selon la revendication 8 ou la revendication 9, **caractérisée en ce que** la protection anti-retournement (26) comporte un dispositif diviseur de passage (27) qui est conçu pour diviser un courant de fluide à travers le volume intérieur en au moins deux courants de fluide plus petits.

11. Utilisation de la soupape selon l'une quelconque des revendications précédentes dans un dispositif d'inhalation.

12. Dispositif d'inhalation avec une soupape selon l'une quelconque des revendications 1 à 10.
